# EUROPEAN PATENT APPLICATION

(11) **EP 1 818 362 A1**
(43) Date of publication of application: **15.08.2007**
(21) Application number: 07002619.0
(22) Date of filing: 07.02.2007
(51) Int. Cl.: C08L 5/00, C08L 3/00, C08L 3/02, C08L 33/00, C08J 5/18, A61K 9/70

(54) **Film and film-forming compositions**

(30) Priority: 13.02.2006 US 352959
(71) Applicant: National Starch and Chemical Investment Holding Corporation, New Castle, Delaware 19720 (US)
(72) Inventor: Zhang, Yeli, Hillsborough, NJ 08844 (US); Puri, Reema, Bridgewater, NJ 08807 (US); Siuta-Cruce, Patricia, Montvale, NJ 07645 (US); Foreman, Paul B., Somerville, NJ 08876 (US); Manegold, Todd, Weehawken, NJ 07086 (US)
(74) Representative: Held, Stephan

(57) **Abstract**

The invention provides films and film forming compositions that can be applied onto a tissue surface. The film and the film forming composition comprise at least one polycarboxylated polymer and at least one polysaccharide.

## Description

### Field of the Invention

The invention relates to films and to film forming compositions. The films of the invention adhere to the surface of moist or moistened tissues and erode over time.

### Background of the Invention

Conventional systems used to promote healing of an injury site, to treat a disease state or for delivering an active ingredient to a specific site are designed to achieve a specific desired end result. Some of these delivery systems, such as tablets, capsules, caplets, dissolvable films and the like are designed such that the active is swallowed, after which the active is released via the gastrointestinal tract. When a selected therapeutic agent to be delivered exhibits absorption problems due to solubility limitations, degrades in the gastrointestinal tract, or is extensively metabolized, another method of administration, such as topical administration, transdermal administration or mucosal administration may be chosen. These methods of administration also have their own drawbacks. Topically applied solutions and the like may be washed away by bodily perspiration or may rub off due to mechanical action or the like. Transdermal drug delivery devices provide many advantages, but require a backing support that needs to be removed following use.

There continues to be a need in the art for new and alternative delivery systems for active agents. The present invention addresses this need.

### Summary of the Invention

The present invention provides films and film forming compositions. The formed film acts as a protective barrier and/or is used to deliver an active agent to a desired site.

One embodiment of the invention provides erodible films that comprise at least one polycarboxylated polymer and at least one polysaccharide. One preferred film comprises a crosslinked poly(acrylic acid) and a starch. In one aspect of this embodiment, the film also comprises an active ingredient. Actives that can be delivered using the film of the invention include pharmaceutical, nutraceutical, cosmeceutical and cosmetic agent. If desired, other ingredients, such as for example, plasticizers, emulsifiers, fragrances, humectants, surfactants, colorants, taste masking agents such as a flavor or sweetener, permeation enhancers or other surface modifying agent, and/or the like may be included.

Another embodiment of the invention provides a film forming composition. The film forming composition comprises a mixture of at least one polycarboxylated polymer and at least one polysaccharide dissolved or dispersed in water or in an organic solvent. Preferred film forming agents comprise a crosslinked poly(acrylic acid) and a starch. The film forming composition may also comprise an active ingredient. The active may be a pharmaceutical, a nutraceutical, a cosmeceutical or a cosmetic agent. If desired, other ingredients, such as for example, plasticizers, emulsifiers, humectants, surfactants, fragrances, colorants, taste masking agents such as a flavor or sweetener, permeation enhancers or other surface modifying agent, and/or the like may be included.

In one aspect, the film forming composition is cast onto a substrate surface on which a film is thereafter formed. Following film formation the film may be removed from the substrate and packaged for later application. Alternatively, the film may be packaged along with the substrate for later application, e.g., to traumatized tissue such as a wound, where a backing such as gauze or the like is desirable.

In another aspect, the film forming composition is applied directly onto a tissue surface, such as skin, and thereafter forms a film. The viscosity of the film forming composition can be adjusted for the desired application. The film forming composition may be used, for example, to coat and protect a tissue that is diseased or otherwise wounded and/or to treat a diseased or wounded area.

Still another embodiment of the invention provides a method of applying a film to an individual. The film is applied to a moist tissue surface of the individual. The tissue may be inherently moist, such as when applied to the mucosa, to the surface of an individual's teeth or to damaged tissue such as a serious burn. Alternatively, the tissue, such as a substantially dry area of the skin, can be pre-moistened with water, oil, salve, or other such agent that provides moisture to the site of application. Upon contact with the moist surface, the film adheres to the surface and maintains its adherence. The film comprises at least one polycarboxylated polymer and at least one polysaccharide, and preferably will also comprise a pharmaceutical, a nutraceutical, a cosmeceutical or a cosmetic agent. The film will remain adhered until peeled off or eroded or removed by application of a sufficient amount of moisture, e.g., washed away with tap water, or flakes off due to loss of moisture, i.e., desiccation.

Yet another embodiment of the invention provides a method of applying a film forming composition to an individual. The film forming composition comprises at least one polycarboxylated polymer and at least one polysaccharide, and preferably will also comprise a pharmaceutical, a nutraceutical, a cosmeceutical or a cosmetic agent and a carrier liquid such as water. The viscosity of the film forming composition can be adjusted for the desired application. The film forming composition dries over time into a film on the surface to which it was applied.

### Detailed Description of the Invention

### Film

A film is defined herein as a flexible product formed, for example, by casting, extruding or blowing, a flowable composition comprising film forming ingredients, and other optional or desired excipients or actives, and a carrier liquid such as water or other organic solvent onto a substrate surface. Following film formation, the formed film will typically retain at least about 5 wt % up to about 30 wt % moisture. A film will typically but not necessarily have a uniform thickness and will generally vary in thickness from about 0.25 mil to about 125 mils. It will be appreciated that the thickness of a film formed directly onto a tissue surface of an individual can be thinner than 0.25 mil. The film can be formed or processed to have a variety of lengths, widths and shapes depending upon the desired end use application. It will be appreciated that film as defined herein and as understood in the art are different and distinct from thin tablets, which are compressed from solids.

The films of the invention comprise at least one polycarboxylated polymer and at least one polysaccharide. Synthetic polycarboxylated polymers such as polyacrylic acid and sodium carboxymethylcellulose are preferred. Preferred polysaccharides are starches, most preferably a physically, chemically or enzymatically modified starch.

Polycarboxylated polymers particularly useful in the practice of this invention may be modified or unmodified and have a weight average molecular weight of at least 10,000 Daltons, more typically at least about 100,000 Daltons, even more typically above about 1,000,000 Daltons. Modifications may include, but are not limited to cross-linking, neutralization, hydrolysis and partial esterification.

Exemplary polycarboxylated polymers which may be used in the present invention include without limitation poly(acrylic acid), cross-linked poly(acrylic acid), poly(acrylic acid) modified by long chain alkyl acrylates, cross-linked poly(acrylic acid) modified by long chain alkyl acrylates. Typical polycarboxylated polymers of this invention include acrylic acid polymers crosslinked with allyl sucrose, allyl ethers of sucrose, allylpentaerythritol, pentaerythritol or divinyl glycol. Preferred polymers are available from NOVEON under the trade names CARBOPOL^{®}, NOVEON^{®} and PEMULEN^{®}, and from National Starch and Chemical under the trade names STRUCTURE 2001 and 3001. Particularly suitable are the pharmaceutical grades CARBOPOL^{®} 971 P, CARBOPOL^{®} 934P and CARBOPOL^{®} 974P. These examples are not limiting and the polysaccharides according to the present invention may be used in combination with virtually any polycarboxylated polymer.

Useful polysaccharides may be derived from natural products, including plant, animal and microbial sources. Examples of polysaccharides include starch, cellulose, xanthans and gums such as galactomannans. Polysaccharide starches include maize or corn, waxy maize, potato, cassava, tapioca and wheat starch. Other starches include varieties of rice, waxy rice, pea, sago, oat, barley, rye, amaranth, sweet potato, and hybrid starches available from conventional plant breeding, e.g., hybrid high amylose starches having amylose content of 40 % or more, such as high amylose corn starch. Also useful are genetically engineered starches such as high amylose potato and waxy potato starches.

The polysaccharides may be chemically modified or derivatized, such as by etherification, esterification, acid hydrolysis, dextrinization, crosslinking, cationization, heat-treated or enzyme treatment (e.g., with *alpha*-amylase, *beta*-amylase, pullulanase, isoamylase, or glucoamylase). Particularly suitable starches, without limitation, include hydroxyalkylated starches such as hydroxypropylated or hydroxyethylated starches, and succinated starches such as octenylsuccinated or dodecylsuccinated starches. The hydroxyalkylated starches have the added advantage of forming a softer film so that there is less or no need for a plasticizer. Also preferred starches are low amylose starches. As used herein, the term "low amylose" is intended to include starches containing less than 40 % by weight amylose. One preferred type of starches is hydroxypropylated starch available from National Starch and Chemical Company. Other preferred starches are waxy starches, also available from National Starch and Chemical Company. As used herein, the term "waxy" is intended to include a starch containing at least about 95 % by weight amylopectin.

The polysaccharides may also be physically modified, e.g., extrusion, spray-dry, drum-dry, agglomeration, pregelatinzation. The starch may be pregelatinized for immediate use in preparing the film forming composition. Alternatively, the starch may be pregelatinized and then stored for later use in preparing the film forming composition. One preferred pregelatinized starch is pregelatinized waxy corn starch, available from National Starch and Chemical Company.

Preferred polysaccharides will have a weight average molecular weight of at least 10,000 Daltons, more preferably at least about 100,000 Daltons, even more preferably above about 500,000 Daltons, and most preferably greater than about 1,000,000 Daltons. While molecular weights of waxy starches are difficult to determine, waxy starches that can be used in the practice of the invention may have weight average molecular weights of 10,000,000 Daltons or more.

Films of the invention will typically comprise from about 0.1 dry weight % to about 95 dry weight % of a polycarboxylated polymer and about 5 dry weight % to about 99.9 dry weight % of a polysaccharide. More preferably, the film will comprise from about 0.1 dry weight % to about 40 dry weight % of a polycarboxylated polymer component and from about 60 dry weight % to about 99.9 dry weight % of polysaccharide.

The film of the present invention may be used to deliver an active component. The film of the invention is used to administer a desired predetermined substance, referred to herein as an "active", an "active ingredient", an "active agent", and the like, at levels sufficient or effective to impart a desired action or specific intended dose. Active components may be added using any of the known methods described in the prior art, and such addition may be carried out during and/or after the production of the film. The active agent may be mixed with the film forming ingredients prior to forming the film or may be dusted onto the surface of the film following its preparation, preferably immediately following casting of the film.

The term active, also referred to herein as an active ingredient, is used to mean any pharmaceutical, nutraceutical, cosmeceutical or cosmetic agent. The active ingredient may or may not be pharmacologically active, but will typically have at least a perceived desired effect.

Pharmaceuticals include prescription, including controlled substances, and over-the-counter drugs.

Non-limiting classes of pharmaceutically active agents include, α-adrenoreceptor agonists, β-adrenoreceptor agonists, α-adrenoreceptor blockers, β-adrenoreceptor blockers, anabolics, analgesics (narcotics and non-narcotics), androgens, anesthetics, antiallergics, antiandrogens, antianginals, antiarrhythmics, antidiabetics, antihistamics, anti-migraine agents, bronchodialators, gestagens and vasodilators. Included are actives for diabetes, antihistamines, pain relief managements, antifungal treatments, hormone managements, sensitive teeth, acne treatments, dental and gum diseases, inflammations, antimicrobial treatments, sedation, insomnia, motion sickness, emesis, nicotine addiction, bladder control and central nervous system diseases.

Specific non-limiting examples of pharmaceuticals include insulin, loratidine, benzocaine, amlexanox, codeine, morphine, nicotine, fentanyl, miconazole, estradiol, progesterone, testosterone, potassium nitrate, salicylic acid, benzoyl peroxide, triclosan, fluoride, chorhexidine, diclofenac, ketoprophen, lidocaine and hydrocortisone.

Nutraceuticals are defined as any substance that may be considered as a food or part of food and provides medical and health benefits, including prevention and treatment of disease.

Types of nutraceutical actives that may be administered include, but are not limited to, functional foods, dietary supplements, herbal products, including anti-oxidants, immune enhancers, cardiovascular health enhancers, healthy joints and cartilage enhancers, memory and mental enhancers, women's health enhancers, mood and emotional enhancers, and weight loss enhancers.

Non-limiting examples of nutraceuticals include ginger, lutein, garlic, lycopene, capsaicin, caffeine, folic acid, beta-carotene, lycopene, valerian, ginseng, vitamin E, herbal teas (e.g., green tea) and natural biological flora.

Cosmeceuticals are defined as any substance or product for topical application and intended to have therapeutic effects on the body.

Types of cosmeceutical actives that may be topically administered include, but are not limited to, moisturizers, anti-aging agents, antioxidants, self tanners, depigmenting agents, scar managements, scalp treatments, enzymes, UV blockers (both organic and inorganic), antiperspirants, hormone creams and amino acids.

Non-limiting examples of cosmeceuticals include alpha and beta hydroxy acids, glycolic acid, lactic acid, antioxidants, vitamin C, vitamin E, botanicals, papain enzyme, hydroquinone, kojic acid, thioglycolate, octylmethoxycinnamate; titanium dioxide, aluminum chlorohydrate, oatmeal and orange peel.

Cosmetic agents are defined as substance or product intended to be applied to an individual for cleansing, beautifying, promoting attractiveness, or altering the appearance. Included are actives for skin creams, lotions, perfumes, lipsticks, fingernail polishes, eye and facial make-ups, shampoos, permanent waves, hair colors and components of cosmetic products.

Types of cosmetic agents that may be administered include, but are not limited to, pigments, dyes, fixatives, emollients, moisturizers, fillers, fragrances, cleansing agents (including both surfactant and abrasive types), moisturizers, conditioning agents, deodorants and shine enhancers.

Non-limiting examples of cosmetics agents include iron oxides, p-phenylenediame, octylacrylamide, acrylates, butylaminoethyl, methacrylate copolymer, petrolatum, hydroxyalkylurea, talc, limonene, sodium lauryl sulfate, silica, polyquarternium-10, musk and silicones.

It is to be understood that the above examples, including classification, are for purpose of illustration only. It will be recognized that all countries do not classify and/or regulate actives in a consistent manner. An active that treats or prevents disease, or otherwise affects the structure and/or function of the individual, may be considered to be a pharmaceutical by one country, but considered to be a cosmeceutical by another. Likewise, variations in classification exist on the cultural level as well as the individual level. What one individual may regard as a cosmeceutical, another may regard as a cosmetic agent.

Active loading levels for active-containing films of the invention will generally range from about 0.01 dry weight % to about 80 dry weight %, more typically from about 0.1 dry weight % to about 70 dry weight %, even more typically from about 1 dry weight % to about 60 dry weight %. It will be appreciated that active loading will depend on, e.g., the type of active, the size and the thickness of the film used, the individual to which the film is to be applied (human adult or child, non-human animal), etc.

In the practice of the invention, the active is solubilized or dispersed in an aqueous environment at or above room temperature. The active may be first solubilized or dispersed in water, and then the active-containing solution or suspension is mixed with the film forming ingredients to form a mixture. Alternatively, and more preferably, the active may be solubilized or dispersed in a solution or suspension of film forming ingredients to form a mixture. The mixture is then coated onto a suitable substrate to form a film and then dried to a moisture content of less than about 15 weight % moisture, more typically from about 5 weight % to about 15 weight % moisture, even more typically from about 6 weight % to about 10 weight % moisture. The formed film comprising the active substance can be air-dried or dried under warm air. The film may then be cut to the desired dimension, packaged and stored.

The film is applied to a moist tissue surface of the individual. The tissue may be inherently moist, such as when applied to the mucosa, to the surface of an individual's teeth or to damaged tissue such as a serious burn. Alternatively, the tissue, such as a substantially dry area of the skin, can be pre-moistened with water, oil, salve, or other such agent that provides moisture to the site of application. Upon contact with the moist surface, the film adheres to the surface and maintains its adherence. The film will remain adhered until eroded by application of a sufficient amount of moisture, e.g., washed away with tap water, or such later time when the film is otherwise removed, e.g., due to loss of complete moisture.

Tissue is used broadly herein to mean any exposed surface of an individual. By exposed surface is meant any area of the body that can be reached without an invasive (e.g., surgical) procedure. Such tissues include but are not limited to skin, various mucosal tissues (mouth, vaginal, nasal, ocular tissue, rectal), keratin (nail, hair) and teeth.

The term "individual" is used herein in its broadest sense and includes animals (both human and non-human, including companion animals such as dogs, cats and horses, and livestock such as cattle and swine) and plants (both agricultural and horticultural applications).

The active is added in such amounts that the final active-containing single dosage form comprises a pre-determined effective amount. By effective amount is meant that the active agent is present in amounts required to impart a desired action or therapeutic dose. The active is present in an amount sufficient, also referred to herein as an effective amount, to bring about a desired result, e.g., a desired therapeutic result in the treatment of a condition. An effective amount of a drug, for example, means a nontoxic but sufficient amount of a drug to provide the selected effect over a specific predetermined period of time or number of doses. It will be appreciated that an amount that constitutes an effective amount will vary according to the particular active incorporated in the film, the condition and/or severity of the condition being treated, any other actives being co-administered with the selected active, desired duration of treatment and preferred number of dosage units, age of the individual to which the film is being administered, and the like. In this regard it is common in the medicinal art that one or two administrations are recommended, e.g., depending on body weight, age, or the like, for administration as a single dose.

The film may be used for both systemic and local administration of the active ingredient. The film may be used for immediate or otherwise controlled release of the active. Controlled release, as used herein, is intended to mean a method and composition for making an active ingredient available to the biological system of a host. Controlled release includes the use of instantaneous release, delayed release, and sustained release. "Instantaneous release" refers to immediate release to the biosystem of the host. "Delayed release" means the active ingredient is not made available to the host until some time delay after administration. "Sustained Release" generally refers to release of active ingredient whereby the level of active ingredient available to the host is maintained at some level over a period of time. The method of affecting each type of release can be varied.

The films may also contain other optional or desired components such as, without limitation, plasticizers, emulsifiers, humectants, surfactants, gelling agent, colorants, taste masking agents such as a flavor or sweetener, permeation enhancers or other surface modifying agents, and fragrances.

It will be appreciated that flavors and/or sweeteners in one embodiment may be an active, while a flavor and/or sweetener in other embodiment may be used, e.g., as a masking agent. The amount of flavoring employed is normally a matter of preference subject to such factors as flavor type, individual flavor, strength desired and taste masking required. Thus, the amount may be varied in order to obtain the result desired in the final product. Such variations are within the capabilities of those skilled in the art without the need for undue experimentation. These optional components are typically added in minor amounts, particularly less than about 35 dry weight % of the film.

### Film forming composition

The film forming composition used to manufacture the films of the invention may also be directly applied to a tissue surface of an individual. The composition may be used in the form of, e.g., a solution, suspension, emulsion, or colloidal dispersion. Non-limiting examples of the film forming compositions that can be directly applied to the tissue include gels, solutions, aerosols and the like. The final form of the composition can be obtained by controlling e.g., the viscosity of the film forming composition and excipients used.

The film forming composition will be applied to a substantially dry tissue surface of an individual and dries naturally into a flexible film when maintained in a substantially dry environment.

The film forming composition comprises at least one polycarboxylated polymer and at least one polysaccharide in water or an organic solvent. Particularly preferred is water. Examples of organic solvents include ethanol and dimethyl sulfoxide.

The same polycarboxylated polymer and polysaccharide as described previously for the film may be used for the film forming composition. Preferred film forming compositions comprise a physically, chemically or enzymatically modified starch and a synthetic polycarboxylated polymer.

The film forming composition of the invention may also comprise an active such as a pharmaceutical, a nutraceutical, a cosmeceutical, a cosmetic agent. The same actives previously used in the film embodiments of the invention may be used in film forming embodiments of the invention.

Other optional components, as described for the film, such as plasticizers, emulsifiers, humectants, surfactants, colorants, taste masking agents such as flavors or sweeteners, permeation enhancers or other surface modifying agents, fragrances and/or the like may be included to the film forming compositions.

### Application of the Film to a Tissue Surface

The film may be used to deliver a therapeutically effective amount of an active to a desired tissue site of an individual such as skin, various mucosal tissues, keratinous tissues and teeth. The film can be applied to healthy tissues, or to damaged or irritated or diseased tissue surfaces. The film has a flexible characteristic which allows the film to remain on the directed site until fully eroded or otherwise removed.

For application onto a moist surface, such as the mucosa, the film will adhere to the moist tissue surface where it releases an active. For application onto a dry tissue surface, sufficient moisture is first applied before the application of the film onto the desired tissue site. Moisture is more typically but not necessarily added to the substrate to which the film is to be applied. The moisture may be, for example, water, saliva, blood, sweat, bodily fluid, lotion or oil.

A film comprising an active may be used to deliver the active to the targeted tissue site. A film, with or without an active component may be applied over a tissue site that has been pre-treated with an active, in order to protect the site and/or provide a combination of actives.

The film may be applied onto a desired site, and will remain as a film on the site until it is peeled off, fully eroded by sufficient moisture or by desiccation or removed with tap water or other solvent.

The film is well suited for the delivery of a wide range of active ingredients via the mucous membranes of a patient, particularly the buccal mucosa. Therapeutic agents that exhibit absorption problems due to solubility limitations, erosion in the gastrointestinal tract, or extensive metabolism are particularly well suited for this mode of administration.

The film remains adhered onto the directed site until eroded by application of sufficient amount of moisture. In a substantially wet environment, the film is removed by natural erosion at the directed site. It will be appreciated that components of the film can be selected to control the time and degree or extent of erosion over time, erosion may take over several seconds, minutes, hours or even days. The dimensional sizes, shape, thickness, and weight of the film will vary depending on the purpose, the site of delivery, and the individual (e.g., human, dog, horse) being treated. The erosion of the film is also determined by the nature of the solubility and the molecular weight of the polymer and starch, the size, the thickness, the density of the film, the active and additive content, and the moisture content of the site, e.g., higher moisture content increases the erosion rate.

The film, if no longer desired, may be easily removed. The film may be removed by peeling it off or washing it away with water. The film may also flake off due to erosion over time accompanied by a substantial loss of moisture.

### Application of the Film forming composition

Similar to the film of the invention, the film forming composition may be directly applied onto the targeted tissue site. Non-limiting examples of the film forming composition may be in the form of a gel, solution and/or an aerosol. Other aqueous mixtures are contemplated by controlling the viscosity of the final systems of the product.

If an active has been incorporated in the film forming composition, the film forming composition may be used to deliver the active to the targeted tissue site. The film forming composition, with or without an active may further be administered over a tissue site that has been pre-treated with an active. The film forming composition will dry into a flexible film at the site of application. Moisture will typically be removed naturally by exposure to air, but drying may be facilitated by use of artificially means, e.g., radiation or heat. The dried film remains adhered on the applied site until it is fully eroded or otherwise removed.

Erosion of the films formed using the film forming compositions of the invention may occur over the course of seconds, minutes, hours or even days depending on the environment. It will be appreciated that components of the film forming aqueous mixture can be selected to control the time and degree or extent of erosion over time as well as the coating thickness, and weight of the film formed, and the moisture content of the site.

The invention will be described further in the following examples, which are included for purposes of illustration and are not intended, in any way, to be limiting of the scope of the invention.

### EXAMPLES

### EXAMPLE 1

### Films and Film Forming Components

Table I lists the components and their respective amounts and dry weight percents to form a Comparative Film A and Film 1. All ingredients were weighed out and blended at room temperature. For the Comparative Film A, the hydroxypropylated tapioca starch was dissolved in water. Glycerin was then added and this mixture was blended in a Waring Commercial Laboratory Blender, model 34BL97, for 3-5 minutes at speed three until the solution became homogenous.

Film 1 was made using a liquid mixture method. For the liquid mixture method, hydroxypropylated tapioca starch was dissolved in water and then combined with 3% solution of Carbopol® 974P to form a starch-polymer solution. After a smooth mixture of the starch-polymer was formed, the rest of the components were added and blended in a Waring Commercial Laboratory Blender, model 34BL97, for 3-5 minutes at speed three until the solution became homogenous.

**Table I**

| Components | Comparative Film A (g) | Comparative Film A (dry wt %) | Film 1 (g) | Film 1 (dry wt %) |
|---|---|---|---|---|
| Hydroxypropylated tapioca starch¹ | 10.0 | 87.0 | 10.0 | 78.3 |
| Carbopol^{®} 974P² diluted to 3% solution | 0 | 0 | 42.6 | 10.0 |
| Glycerin³ | 1.5 | 13.0 | 1.5 | 11.7 |
| Deionized Water | 26.8 | | 41.3 | |

| | | | | |
|---|---|---|---|---|
| ¹ National Starch & Chemical ² Noveon, Inc. ³ Spectrum | | | | |

Table II lists the components and their respective amounts and dry weight percent to create a two films and a film forming composition. Film 2 was also made using the liquid mixture method. Pregelatinized waxy corn starch and Carbopol^{®} 974P were separately dissolved in water and then combined to form a starch-polymer solution. After a smooth mixture of the starch-polymer was formed, the rest of the components were added and blended in a Waring Commercial Laboratory Blender, model 34BL97, for 3-5 minutes at speed three until the solution became homogenous.

Film 3 was made using a co-spray dry method. For the co-spray dry method, a solution mixture of pregelatinized waxy corn starch and Carbopol^{®} 974P were first dissolved in water and then co-sprayed. The co-spray dried mixture was then re-dispersed in water to form a starch-polymer solution. After a smooth mixture of the starch-polymer was formed, the rest of the components were added and blended in a Waring Commercial Laboratory Blender, model 34BL97, for 3-5 minutes at speed three until the solution became homogenous.

The film forming composition was also made using the co-spray dry method. After redispersing the starch-polymer mixture in water, the rest of the components were added and blended in a Waring Commercial Laboratory Blender, model 34BL97, for 3-5 minutes at speed three until the solution became homogenous.

**Table II**

| Components | Film 2 (g) | Film 2 (dry wt %) | Film 3 (g) | Film 3 (dry wt %) | Film Forming Composition (g) | Film Forming Composition (dry wt %) |
|---|---|---|---|---|---|---|
| Pregelatinized waxy corn starch¹ | 15.9 | 76.8 | 9.4 | 50.0 | 10.0 | 37.4 |
| Carbopol^{®} 974P² | 1.6 | 7.7 | 3.1 | 16.5 | 3.3 | 12.4 |
| Glycerin³ | 3.2 | 15.5 | 3.8 | 20.2 | | |
| Ethanol³ | | | | | 6.7 | 25.1 |
| Neosporin⁴ | | | 2.5 | 13.3 | | |
| Aveeno Lotion⁵ | | | | | 6.7 | 25.1 |
| Deionized Water | 79.3 | | 81.2 | | 73.3 | |

| | | | | | | |
|---|---|---|---|---|---|---|
| ¹ National Starch & Chemical ² Noveon, Inc. ³ Spectrum ⁴ Pfizer ⁵ Johnson & Johnson | | | | | | |

### Example 2

### Film Casting

To cast a film, 10 cc of Film 2 solution was withdrawn with a syringe. The solution was then ejected onto the coater to cast a film on a PET substrate. The wet film was initially cast at 2 mils thickness. The film on the substrate was then dried in a 150°F convection oven for 5-8 minutes, or until not tacky to touch. The final film dried to 0.7 mil thickness. This was cut into appropriate dimensions, packed, and stored.

### Example 3

### Dissolution Rate of Comparative Film A and Film 1 in Water

The Comparative Film A and Film 1 were prepared according to Example 1 with dimensions of 1" x 1.25" x 0.003." Both films were both placed in a beaker of water. It took approximately 54 seconds before any visible erosion of the Comparative Film A was evident. For Film 1, approximately 3.5 minutes elapsed before any visible erosion of the film was evident. Film 1 with the same dimensions was placed atop of the water. It took approximately 22 minutes before any visible erosion of Film 1 was evident.

### Example 4

### Application of Film onto a Mucosal Surface

Film 2 prepared by Example 1 with dimensions of 1" x 1.25" x 0.0007" was placed inside the mouth of a volunteer, on the buccal surface tissue (inner cheek surface). The film adhered instantaneously onto the buccal surface. The film remained adhered onto the buccal surface until completely dissolved. The erosion took approximately 25 seconds.

### Example 5

### Application of Film onto Wet Skin Surface

Film 2 prepared by Example 1 with dimensions of 1" x 1.25" x 0.0015" was placed onto a wet skin surface. The film adhered instantaneously onto the wet surface of the skin. A slow continuous stream of tap water was applied onto the film. The stream of tap water was kept at a minimum to avoid shearing of the film. The film remained adhered onto the wet skin surface until completely eroded. The erosion took approximately 30 minutes.

### Example 6

### Application of Film onto Dry Skin Surface

Two to three drops of tap water was applied to the back of a dry hand. Film 3 prepared by Example 1 with dimensions of 1" x 1.25" x 0.0015" was placed onto the wetted area of the hand. The film adhered instantaneously onto the wetted skin surface. The film remained adhered onto the wetted skin surface until the residue was washed off three hours later.

### Example 7

### Application of Film Forming Composition onto Skin Surface

Approximately 1.5 g of the Film Forming Composition, as prepared by Example 1, was applied onto the back of a dry hand and rubbed onto the skin until transparent on the skin surface. The Film Forming Composition dried onto the skin surface into a flexible film. The film remained on the skin surface until washed with running water.

Many modifications and variations of this invention can be made without departing from its spirit and scope, as will be apparent to those skilled in the art. The specific embodiments described herein are offered by way of example only, and the invention is to be limited only by the terms of the appended claims, along with the full scope of equivalents to which such claims are entitled.

## Claims

1. A film comprising at least one polycarboxylated polymer and at least one polysaccharide.

2. The film of claim 1 further comprising an active.

3. The film of claim 2 wherein the active is a pharmaceutical agent.

4. The film of claim 1, wherein the polycarboxylated polymer is a crosslinked poly(acrylic acid).

5. The film of claim 4, wherein the polysaccharide is a starch.

6. A film forming composition comprising at least one polycarboxylated polymer and at least one polysaccharide in water or other solvent.

7. The film forming composition of claim 6 further comprising an active.

8. The film forming composition of claim 7 wherein the active is a pharmaceutical agent.

9. The film forming composition of claim 8, wherein the polycarboxylated polymer is a crosslinked poly(acrylic acid).

10. The film forming composition of claim 9, wherein the polysaccharide is a starch.

11. A method of delivering an active to an individual comprising applying the film of claim 1 to a moist tissue surface of said individual whereby the film adheres to said moist surface.

12. The method of claim 11 wherein said film is applied over a pre-applied active.

13. The method of claim 11 wherein said film comprises of an active.

14. The method of claim 11 wherein the film is applied onto a premoistened tissue surface.

15. The method of claim 11 wherein the film is applied onto a mucosal surface.

16. A method of applying a film to a tissue surface of an individual comprising applying the film forming composition of claim 6 to a tissue surface of an individual and allowing the composition to form a film.

17. The method of claim 16 wherein said film forming composition is applied over a pre-applied active.

18. The method of claim 16 wherein said film forming composition comprises an active.

19. A method of forming a film comprising preparing a solution of at least one polycarboxylated polymer and at least one polysaccharide and coating the solution onto a substrate to form a film.

20. The method of claim 19 wherein the solution is coated onto the surface of an individual's skin.
